# EUROPEAN PATENT APPLICATION

(11) **EP 1 803 824 A1**
(43) Date of publication of application: **04.07.2007**
(21) Application number: 06256496.8
(22) Date of filing: 21.12.2006
(51) Int. Cl.: C12Q 1/68

(54) **SNP discrimination assay, and DNA chips for SNP discrimination**

(30) Priority: 28.12.2005 JP 2005379376
(71) Applicant: SONY CORPORATION, Tokyo 141 (JP)
(72) Inventor: Negishi, Makiko, c/o Sony Corporation, Tokyo 141 (JP)
(74) Representative: Jackson, Jonathan Andrew

(57) **Abstract**

Disclosed herein is an SNP discrimination assay for discriminating base species of an SNP in a specific gene in a sample, which includes the steps of hybridizing four detection probes, which have at least a flanking sequence around an SNP in the gene and are different in base species at a site of the SNP, with a target nucleic acid in the sample, the target nucleic acid having at least the flanking sequence around the SN in the gene, respectively, cleaving three of four double-stranded nucleic acids, which contain noncomplementary base pairs, respectively, at sites of the noncomplementary base pairs, denaturing only the three double-stranded nucleic acids, which have been cleaved at the sites of the noncomplementary base pairs, into single-stranded forms, and detecting one of the four detection probes, the one detection probe still retaining a double-stranded form after the nucleic acid denaturation step.

## Description

This invention relates to an SNP discrimination assay for discriminating the base species of an SNP (single-base polymorphisms) in a specific gene, and also to DNA chips for SNP discrimination.

More specifically, the present invention is concerned with an SNP discrimination assay including inter alia a step of hybridizing four detection probes, which are different in base species at a site of an SNP, with a target nucleic acid, respectively, a step of cleaving resultant double-stranded nucleic acids at sites of noncomplementary base pairs and a step of denaturing only double-stranded nucleic acids, which have been cleaved at the sites of the noncomplementary base pairs, into single-stranded forms; and also with a DNA chip for SNP discrimination, which has four detection probes different in base species at a site of an SNP and separately held or immobilized in different reaction regions, respectively.

The term "single nucleotide polymorphisms (SNP)" means a state that the base sequence of a gene is different at only one site, and the site. SNPs are gathered to exist at a frequency of one site over every several hundreds of bases in 3 billion human DNA bases, and further, differences in SNP base species are presumed to determine the predisposition or the like of individuals.

Accordingly, a functional analysis of SNPs may possibly make it possible to predict susceptibility to a particular disease, reactivity to a specific medicine, or the like. SNP analysis is, therefore, expected to find applications to so-called tailor-made remedy.

As a method for discriminating base species of SNPs, the arrayed primer extension (APEX) method has been proposed, for example. According to the APEX method, subsequent to hybridization of a detection probe, which has a sequence up to one site before an SNP site, with a target nucleic acid, the detection probe is caused to extend by only one base with a DNA polymerase in the presence of a deoxynucleotide modified with four-color fluorescent substance, thereby introducing the fluorescence-modified deoxynucleotide into a free end (SNP site) of the detection probe. By fluorescently exciting the deoxynucleotide introduced in the detection probe, the base species of the SNP can be discriminated.

As illustrative documents relating to SNP detections, Japanese Patent Laid-Open No. 2004-357570 discloses a method for detecting the existence/non-existence of a mismatch pair by an electrochemical technique, and Japanese Patent Laid-Open No.2001-242135 discloses a method for detecting an SNP or the like by using pin electrodes.

The discrimination of base species of an SNP, for example, with a DNA chip or the like is accompanied by a problem in that the accuracy of detection can be hardly increased, although it permits a high through-put analysis. It is desirable to provide a novel method, which can promise increased detection accuracy upon discrimination of base species of an SNP and further, can be applied to DNA chips and the like.

In one embodiment of the present invention, there is thus provided an SNP discrimination assay for discriminating base species of an SNP in a specific gene in a sample, which includes at least the steps of: (1) hybridizing four detection probes, which have at least a flanking sequence around an SNP in the gene and are different in base species at a site of the SNP, with a target nucleic acid in the sample, the target nucleic acid having at least the flanking sequence around the SN in the gene, respectively; (2) cleaving three of four double-stranded nucleic acids, which have been formed by the hybridization step and contain noncomplementary base pairs, respectively, at sites of the noncomplementary base pairs, preferably by using an enzyme capable of specifically cleaving single-stranded nucleic acid; (3) denaturing only the three double-stranded nucleic acids, which have been cleaved at the sites of the noncomplementary base pairs, into single-stranded forms; and (4) detecting one of the four detection probes, the one detection probe still retaining a double-stranded form after the nucleic acid denaturation step.

Provided firstly is the four detection probes, which have at least the flanking sequence around the SNP in the gene and are different in base species at a site of the SNP. Then, the four detection probes and the target nucleic acid are hybridized, respectively, to form the four double-stranded nucleic acids. Upon this hybridization, with one of the four detection probes, the base pair at the SNP site becomes complementary, and with each of the remaining three detection probes, the base pair at the SNP site becomes noncomplementary (mismatched).

Among the resultant four double-stranded nucleic acids, the three double-stranded nucleic acids which contain noncomplementary base pairs, respectively, are next cleaved at the sites of the noncomplementary base pairs, preferably by using an enzyme capable of specifically cleaving single-stranded nucleic acid. As a result, in each of the three detection probes each containing the noncomplementary base pair at its SNP site, the double-stranded nucleic acid is cleaved at the SNP site. In the remaining one detection probe containing the complementary base pair at its SNP site, on the other hand, the SNP site is retained without cleavage.

Subsequently, only the three double-stranded nucleic acids which have been cleaved at the sites of the noncomplementary base pairs are denatured into the single-stranded forms, for example, by making use of differences in melting temperature (hereinafter abbreviated as "Tm") between remaining one of the four double-stranded nucleic acids, the remaining one double-stranded nucleic acid having not been cleaved in the mismatch site cleavage step, and the double-stranded nucleic acids cleaved at the sites of the noncomplementary base pairs in the mismatch site cleavage step. The detection probe, which still retains the double-stranded form after the denaturation of the mismatch nucleic acids into the single-stranded forms, is then detected, preferably by using an intercalator capable of specifically binding to double-stranded nucleic acids.

When the SNP site of a detection probe is mismatched upon hybridization, the detection probe is denatured into a single-stranded form in a nucleic acid denaturation step so that a substance (intercalator or the like) capable of specifically binding to double-stranded nucleic acids does not bind to the denatured detection probe. When the SNP of a detection probe is complementary (in the case of a perfect match), on the other hand, the double-stranded form is retained even after the nucleic acid denaturation step. Accordingly, the substance (intercalator or the like) capable of specifically binding to double-stranded nucleic acids binds to the undenatured detection probe.

As is appreciated from the foregoing, the one of the four detection probes, the one detection probe being complementary at its SNP site at the time of the hybridization, can be detected by the above-described method. It is, therefore, possible to discriminate the base species of an SNP relating to a specific gene in a biological sample.

It is to be noted that the assay according to an embodiment of the present invention can promise higher detection accuracy as an advantage because the base species of an SNP are not discriminated by hybridization at the SNP site but the SNP site are discriminated by an enzyme capable of specifically recognizing mismatch sites. In other words, the higher detection accuracy is available because the reaction specificity of an enzyme to a nucleic acid containing a noncomplementary base pair is more distinctive than a difference between the intermolecular attractive force (i.e., a difference in attractive force by hydrogen bonds between molecules) of a nucleic acid containing a complementary base pair and the intermolecular attractive force of a nucleic acid containing a noncomplementary base pair.

The SNP discrimination assay according to an embodiment of the present invention can be practiced, for example, with a DNA chip carrying thereon four detection probes, which have at least a flanking sequence around an SNP in a gene, are different in base species at a site of the SNP, and have been held or immobilized beforehand on a surface of a substrate.

According to an embodiment of the present invention, the base species of SNPs in various genes can be discriminated with high accuracy. In addition, the present invention can also be applied to DNA chips and the like, thereby enabling high throughput, comprehensive and high accuracy discrimination of SNPs. Therefore, the present invention is expected to determine the predisposition or the like of individuals or to predict susceptibility to a particular disease, reactivity to a specific medicine, or the like. In other words, the present invention is expected to find applications to so-called tailor-made remedy.

Various respective aspects and features of the invention are defined in the appended claims. Features from the dependent claims may be combined with features of the independent claims as appropriate and not merely as explicitly set out in the claims.

The invention will now be described by way of example with reference to the accompanying drawings, throughout which like parts are referred to by like references, and in which:
FIG. 1A is a diagram showing variations in the resonance frequency of a quartz resonator upon addition of a target nucleic acid to a reaction bath when (perfect match) nucleic acids, base pairs of which were all complementary, were used;
FIG. 1B is a diagram showing variations in the resonance frequency of a quartz resonator upon addition of "E. COLI ENDONUCLEASE V" (trade name for an enzyme capable of recognizing mismatch sites, product of TREVIGEN, INC.) to the reaction bath when the (perfect match) nucleic acids, the base pairs of which were all complementary, were used;
FIG. 1C is a diagram showing variations in the resonance frequency of a quartz resonator upon addition of RecA to the reaction bath when the (perfect match) nucleic acids, the base pairs of which were all complementary, were used;
FIG. 2A is a diagram showing variations in the resonance frequency of a quartz resonator upon addition of the target nucleic acid to the reaction bath when A-G mismatch nucleic acids were used;
FIG. 2B is a diagram showing variations in the resonance frequency of a quartz resonator upon addition of "E. COLI ENDONUCLEASE V" to the reaction bath when the A-G mismatch nucleic acids were used;
FIG. 2C is a diagram showing variations in the resonance frequency of a quartz resonator upon addition of RecA to the reaction bath when the A-G mismatch nucleic acids were used;
FIG. 3A is a diagram showing variations in the resonance frequency of a quartz resonator upon addition of the target nucleic acid to the reaction bath when T-T mismatch nucleic acids were used;
FIG. 3B is a diagram showing variations in the resonance frequency of a quartz resonator upon addition of "E. COLI ENDONUCLEASE V" to the reaction bath when the T-T mismatch nucleic acids were used;
FIG. 3C is a diagram showing variations in the resonance frequency of a quartz resonator upon addition of RecA to the reaction bath when the T-T mismatch nucleic acids were used;
FIG. 4A is a diagram showing variations in the resonance frequency of a quartz resonator upon addition of the target nucleic acid to the reaction bath when T-G mismatch nucleic acids were used;
FIG. 4B is a diagram showing variations in the resonance frequency of a quartz resonator upon addition of "E. COLI ENDONUCLEASE V" to the reaction bath when the T-G mismatch nucleic acids were used;
FIG. 4C is a diagram showing variations in the resonance frequency of a quartz resonator upon addition of RecA to the reaction bath when the T-G mismatch nucleic acids were used;
FIG. 5A is a diagram showing variations in the resonance frequency of a quartz resonator upon addition of the target nucleic acid to the reaction bath when T-C mismatch nucleic acids were used;
FIG. 5B is a diagram showing variations in the resonance frequency of a quartz resonator upon addition of "E. COLI ENDONUCLEASE V" to the reaction bath when the T-C mismatch nucleic acids were used;
FIG. 5C is a diagram showing variations in the resonance frequency of a quartz resonator upon addition of RecA to the reaction bath when the T-C mismatch nucleic acids were used;
FIG. 6A is a diagram showing variations in the resonance frequency of a quartz resonator upon addition of the target nucleic acid to the reaction bath when G-G mismatch nucleic acids were used;
FIG. 6B is a diagram showing variations in the resonance frequency of a quartz resonator upon addition of "E. COLI ENDONUCLEASE V" to the reaction bath when the G-G mismatch nucleic acids were used;
FIG. 6C is a diagram showing variations in the resonance frequency of a quartz resonator upon addition of RecA to the reaction bath when the G-G mismatch nucleic acids were used;
FIG. 7A is a diagram showing variations in the resonance frequency of a quartz resonator upon addition of the target nucleic acid to the reaction bath when C-C mismatch nucleic acids were used;
FIG. 7B is a diagram showing variations in the resonance frequency of a quartz resonator upon addition of "E. COLI ENDONUCLEASE V" to the reaction bath when the C-C mismatch nucleic acids were used;
FIG. 7C is a diagram showing variations in the resonance frequency of a quartz resonator upon addition of RecA to the reaction bath when the C-C mismatch nucleic acids were used;
FIG. 8 is a diagram showing variations in the resonance frequency of a quartz resonator when A-A mismatch nucleic acids were used and the temperature of the reaction bath was set at 25°C;
FIG. 9 is a diagram showing variations in the resonance frequency of a quartz resonator when A-C mismatch nucleic acids were used, the temperature of the reaction bath was set at 37°C, and a mismatch site of a detection probe was set at the seventh site down from a top (i.e., an end on a side opposite to a fixed end); and
FIG. 10 is a diagram showing variations in the resonance frequency of a quartz resonator when G-T mismatch nucleic acids were used and a mismatch site of a detection probe was set at the seventh site up from a bottom (i.e., a fixed end).

### <SNP discrimination DNA chip according to an embodiment of the present invention>

Firstly, a description will hereinafter be made about an illustrative DNA chip to which the present invention has been applied. It should, however, be borne in mind that the SNP discrimination assay according to an embodiment of the present invention is not limited to the use of such a DNA chip.

This DNA chip for SNP discrimination has a substrate with plural reaction regions formed as spots for hybridization on a surface of the substrate. Four detection probes, which have at least a flanking sequence around an SNP in a specific gene and are different in base species at the site of the SNP, are separately held or immobilized in different ones of the reaction regions.

SNP information is obtained from a publicly available database or the like. Based on the information so obtained, the designing of detection probes is conducted.

For the detection probes, a flanking sequence around an SNP in an SNP-containing gene is used. Four detection probes, which contain adenine (A), thymine (T), guanine (G) and cytosine (C), respectively, as base species at the site of the SNP, are synthesized and used. These individual detection probes are separately held or immobilized in the different reaction regions.

Preferably, the SNP site in each detection probe may be located at a position 20 to 35% apart from an end on a side opposite to a fixed end. Described specifically, in the case of a 30-mer detection probe, for example, an SNP site can be the seventh to eighth site from an end opposite to a fixed end. If the SNP site is excessively close to the fixed end, the enzyme which is capable of specifically cleaving single-stranded nucleic acid may not be able to recognize a mismatch at the SNP site in some instances.

An artificial modification such as methylation may be applied to a part of the sequence of each of one or more of the detection probes. By the artificial modification of the part of the sequence, Tm of the detection probe can be adjusted. For example, by applying an artificial modification to each of plural detection probes other than one having the highest Tm such that the plural detection probes other than the one detection probe can be rendered uniform in Tm with the one detection probe, Tm variations in all the detection probes can be reduced. It is, therefore, possible to fabricate a DNA chip which is high in throughput, easy in handling and high in accuracy.

Each detection probe may be designed beforehand such that it can be immobilized on the surface of the substrate via a linker (linking sequence) of a predetermined length to also permit hybridization on the side of its immobilized end. By this designing, each detection probe is immobilized at a position a little apart from the surface of the substrate. It is, therefore, possible to avoid steric hindrance by the surface of the substrate and to further ensure the hybridization. As a consequence, a noise can be reduced upon detection, leading to higher detection accuracy.

No particular limitation is imposed on the method for immobilizing each probe nucleic acid. For example, it is possible to coat the surface of the substrate with streptavidin or the like, to biotinylate ends (fixed ends) of the detection probe and then to immobilize the probe nucleic acid via avidin-biotin bonds. It is also possible to subject the surface of the substrate to surface treatment with thiol (SH) groups, to add thiol groups to ends (fixed ends) of the detection probe and then to immobilize the probe nucleic acid via disulfide bonds (-S-S- bonds).

### <SNP discrimination assay according to an embodiment of the present invention>

A description will next be made about the SNP discrimination assay according to an embodiment of the present invention.

The SNP discrimination assay according to an embodiment of the present invention is conducted using a substrate or the like provided with reaction regions. Separately held or immobilized in different ones of these reaction regions are the four detection probes, which have at least the flanking sequence around the SNP in the specific gene and are different in base species at the site of the SNP. For example, the above-mentioned SNP discrimination DNP chip can be used.

As described above, the SNP discrimination assay according to an embodiment of the present invention includes: (1) a hybridization step of hybridizing four detection probes, which have at least a flanking sequence around an SNP in a specific gene and are different in base species at a site of the SNP, with a target nucleic acid in the sample, the target nucleic acid having at least the flanking sequence around the SN in the gene, respectively; (2) a mismatch site cleavage step of cleaving three of four double-stranded nucleic acids, which have been formed by the hybridization step and contain noncomplementary base pairs, respectively, at sites of the noncomplementary base pairs, preferably by using an enzyme capable of specifically cleaving single-stranded nucleic acid; (3) a nucleic acid denaturation step of denaturing only the three double-stranded nucleic acids, which have been cleaved at the sites of the noncomplementary base pairs, into single-stranded forms; and (4) a base species detection step of detecting one of the four detection probes, the one detection probe still retaining a double-stranded form after the nucleic acid denaturation step.

### (1) Preparation procedure of the target nucleic acid and the hybridization step

Subsequent to preparation of the target nucleic acid, this hybridization step is conducted. Described specifically, the target nucleic acid is added dropwise or otherwise supplied to the reaction regions to hybridize the target nucleic acid with the respective detection probes.

No particular limitation is imposed on the target nucleic acid. For example, a biological sample collected from the human body or the like is used. The preparation of the target nucleic acid can be conducted, for example, by extracting or otherwise isolating a nucleic acid, and then cleaving it into shorter sequences with a restriction enzyme or amplifying a sequence, which is to be subjected to hybridization with the detecting nucleic acids, by PCR or the like. When amplifying the sequence by PCR or the like, a primer has to be designed based on an immediately upstream and/or downstream sequence of the sequence for the detection probes.

Further, an artificial modification such as methylation may be applied to a part of the sequence of the target nucleic acid. Namely, Tm of the target nucleic acid can be adjusted by artificially modifying the part of its sequence.

In the reaction regions which serve as spots for hybridization between the respective detection probes and the target nucleic acid, a buffer solution such as, a phosphate buffer solution, phosphate-buffered physiological saline (PBS), a Goods buffer solution such as HEPES buffer solution, or Tris buffer solution is placed. By adjusting the concentration of the salt (NaCl, MgCl₂ or the like) in the buffer solution, it is possible to avoid nonspecific adsorption of the nucleic acid or the like onto the surface of the substrate. Use of salt-containing HEPES buffer solution or the like also makes it possible to avoid the nonspecific adsorption of the nucleic acid or the like onto the surface of the substrate.

The target nucleic acid is then added dropwise or otherwise supplied into the reaction regions with the buffer solution placed therein to subject the target nucleic acid and the respective detection probes to hybridization.

The temperature in the reaction regions is requisite to be set at such a temperature that subsequent to the hybridization, one of the detection probes, the one detection probe being the lowest in Tm, will not be denatured into a single-stranded nucleic acid.

### (2) Mismatch site cleavage step

In this step, those containing noncomplementary base-pair sites (mismatch sites), respectively, among the double-stranded nucleic acids formed in the hybridization step are cleaved at the mismatch sites, preferably by using an enzyme capable of specifically cleaving single-stranded nucleic acid.

No particular limitation is imposed on the enzyme capable of specifically cleaving single-stranded nucleic acid insofar as it can specifically cleave a phosphate backbone at the site of a noncomplementary base pair in a double-stranded nucleic acid. For example, "E. COLI ENDONUCLEASE V" can be used.

The temperature in the reaction regions is set, for example, within a range of temperatures optimal to the employed enzyme, and further at such a temperature that even the detection probe having the lowest Tm can retain its double-stranded form after the hybridization.

### (3) Nucleic acid denaturation step

In this step, only the nucleic acids which have been cleaved in the mismatch site cleavage step are denatured into single-stranded forms preferably by temperature treatment or the like.

Tm is primarily dependent on the GC content and the nucleotide length. A difference, therefore, arises in Tm between a double-stranded nucleic acid cleaved at a part thereof as a result of cleavage at a mismatch site thereof in the mismatch site cleavage step and a double-stranded nucleic acid not cleaved in the mismatch site cleavage step.

It is, therefore, possible to allow one of the detection probes, the one detection probe having the complementary base pair at the SNP site thereof, to retain its double-stranded form and at the same time, to have the remaining three detection probes denatured (dissociated) into single-stranded nucleic acids, for example, by setting the temperature in the respective reaction regions at a temperature a little lower than Tm of the one detection probe. In other words, only one of the detection probes, the one detection probe having the complementary base pair at the SNP site thereof, can retain the double-stranded form.

### (4) Base species detection step

In this step, the detection probe which still retains the double-stranded form after the nucleic acid denaturation step is detected.

The detection probe which still retains the double-stranded form can be detected with high accuracy, for example, by using an intercalator which specifically binds to a double-stranded nucleic acid. As an alternative example, the nucleic acid which retains the double-bonded form may also be detected by applying fluorescence labeling or the like to the target nucleic acid.

By conducting the above steps, the base species of the SNP in the specific gene can be discriminated. It is to be noted that the present invention is not limited narrowly to only the above-described matter.

### Example 1

In Example 1, verification was conducted primarily as to whether or not site-specific cleavage would take place at all single-base mismatches when a double-stranded nucleic acid is cleaved at the sites of complementary base pairs by using "E. COLI ENDONUCLEASE V" and also as to whether or not the double-stranded nucleic acid would be denatured into a single-stranded form after the cleavage by the enzyme.

As detection probes and a target nucleic acid, 30-mer oligonucleotides were provided, respectively. In the detection probes, the oligonucleotides were biotinylated at one ends thereof. The individual oligonucleotides were custom-synthesized by ESPEC OLIGO SERVICE CORP.

A sequence listing is provided after the claims. The sequence listing shows: in the case that all the base pairs were complementary (perfectly matched), the sequences of a detection probe and a target nucleic acid as SEQ ID NO: 1 and SEQ ID NO: 2, respectively; in the case of an A-G mismatch, the sequences of a detection probe and a target nucleic acid as SEQ ID NO: 3 and SEQ ID NO: 4, respectively; in the case of a T-T mismatch, the sequences of a detection probe and a target nucleic acid as SEQ ID NO: 5 and SEQ ID NO: 6, respectively; in the case of a T-G mismatch, the sequences of a detection probe and a target nucleic acid as SEQ ID NO: 7 and SEQ ID NO: 8, respectively; in the case of a T-C mismatch, the sequences of a detection probe and a target nucleic acid as SEQ ID NO: 9 and SEQ ID NO: 10, respectively; in the case of a G-G mismatch, the sequences of a detection probe and a target nucleic acid as SEQ ID NO: 11 and SEQ ID NO: 12, respectively; in the case of a C-C mismatch, the sequences of a detection probe and a target nucleic acid as SEQ ID NO: 13 and SEQ ID NO: 14, respectively; in the case of an A-A mismatch, the sequences of a detection probe and a target nucleic acid as SEQ ID NO: 15 and SEQ ID NO: 16, respectively; and in the case of an A-C mismatch, the sequences of a detection probe and a target nucleic acid as SEQ ID NO: 17 and SEQ ID NO: 18, respectively. The sequence listing also shows the sequences of a detection probe and a target nucleic acid, which were separately synthesized in the case of a G-T mismatch, as SEQ ID NO: 19 and SEQ ID NO: 20, respectively.

Next, a quartz resonator with its Au surface coated with avidin was provided. As the avidin, "AVIDIN" (trade name, product of Wako Pure Chemical Industries, Ltd.) was used (this will hereinafter apply equally). The coating of the Au surface with avidin was conducted following the procedure described in the protocol of a QCM instrument for the quantitation of biomolecular interactions employed in this experiment ["AFFINIX Q (model: "QCM2000", trade name)", quartz crystal resonation system, resonant frequency: 27 mHz, manufactured by Initium, Inc.; hereinafter referred to as "the QCM instrument"].

The quartz resonator was then placed in a 10 nM solution of the detection probe (SEQ ID NO: 1) to immobilize the detection probe on the quartz resonator via avidin-biotin bonds.

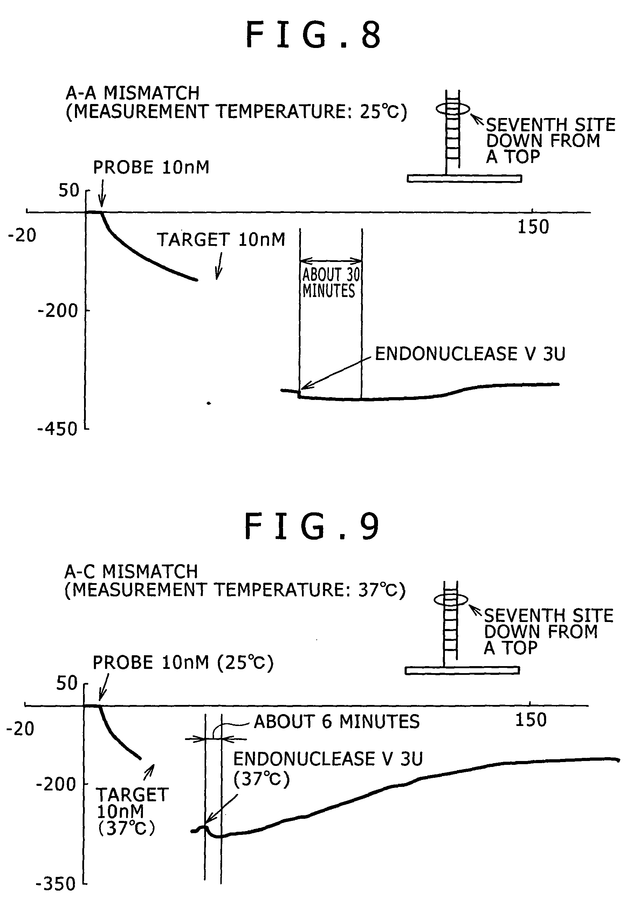

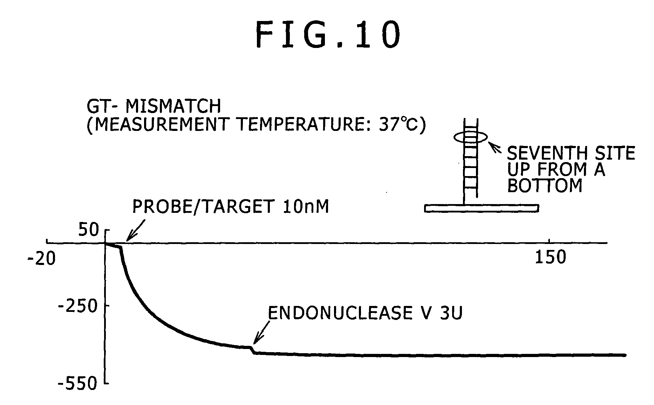

The reaction bath of the QCM instrument was next set at 25°C (at 37°C in experiments on the combinations of SEQ ID NO: 17-SEQ ID NO: 18 and SEQ ID NO: 19-SEQ ID NO: 20, shown in FIGS. 9 and 10), and the quartz crystal was immersed in the reaction bath of the QCM instrument. Subsequently, the target nucleic acid (SEQ ID NO: 2, 10 nM) was added to the reaction bath. Variations (ΔF, unit: Hz; this will hereinafter apply equally) in the resonance frequency of the quartz crystal were measured to determine the amount of a hybridization product between the detection probe, which was immobilized on the crystal resonator, and the target nucleic acid.

"E. COLI ENDONUCLEASE V" (3 units) was next added to the reaction bath of the QCM instrument. Variations in the resonance frequency of the quartz resonator were then measured to determine the amount of a hybridization product between the detection probe, which was immobilized on the crystal resonator, and the target nucleic acid (the amount of the hybridization product still held after the enzyme treatment).

RecA (a protein capable of specifically binding to single-stranded DNAs, 5 µg) was next added to the reaction bath of the QCM instrument. Variations in the resonance frequency of the quartz resonator were then measured to determine the amount of a single-stranded nucleic acid (the amount of the denatured single-stranded nucleic acid after the enzyme treatment).

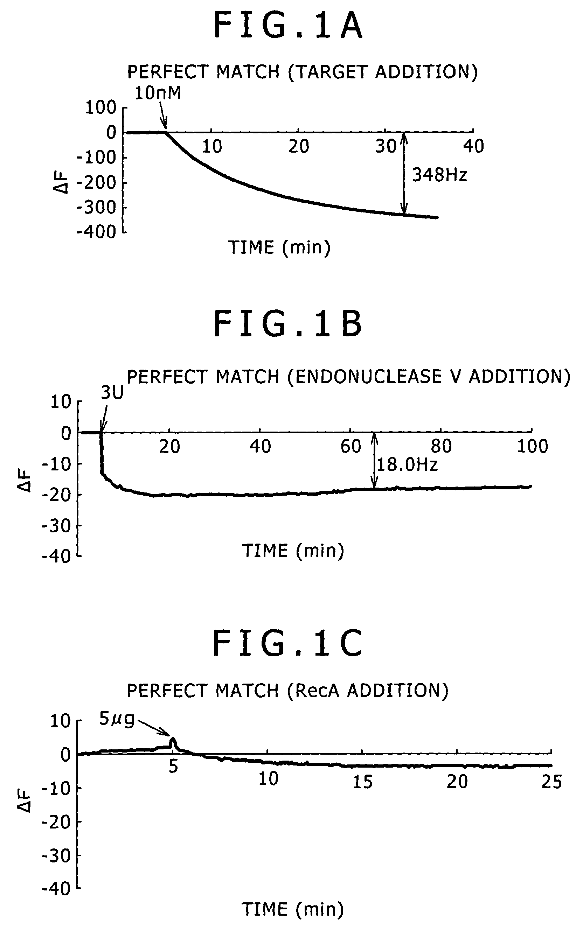

Similar experiments were conducted using the combinations of the remaining detection probes and target nucleic acids (SEQ ID NO: 3-SEQ ID NO: 4 to SEQ ID NO: 19-SEQ ID NO: 20), respectively. The results are shown in FIG. 1A through FIG. 10, in which the abscissa and ordinate of each diagram indicate time (min) and variations (AF,(Hz)) in the resonance frequency of the quartz resonator, respectively.

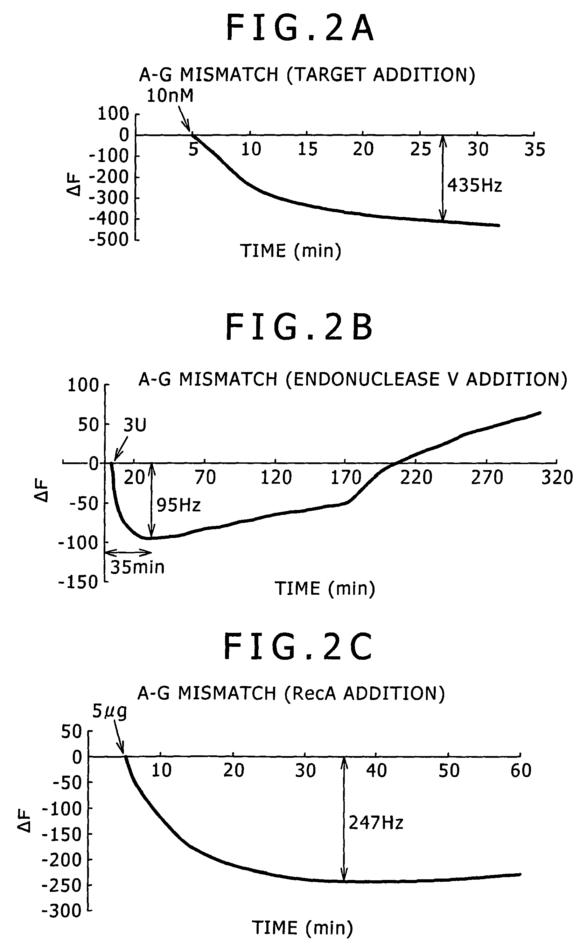

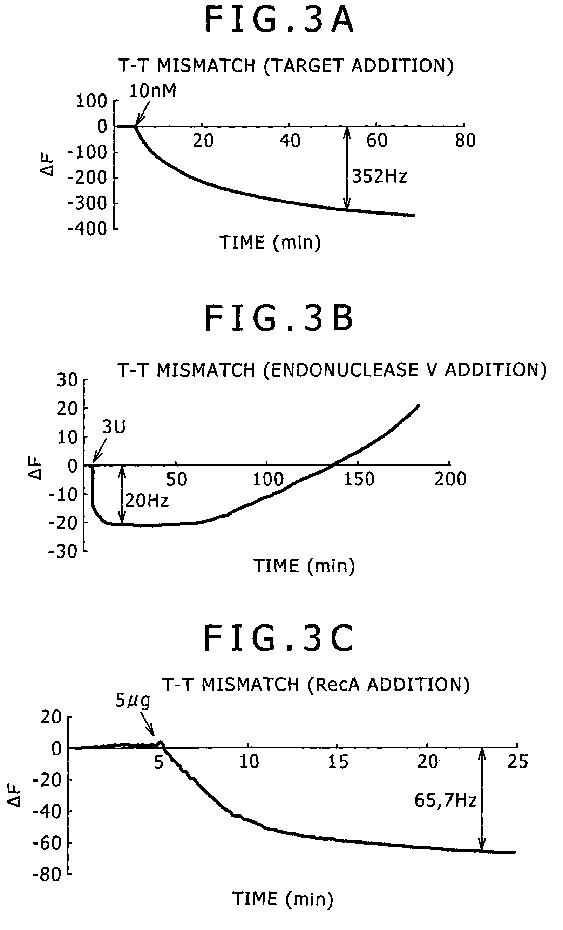

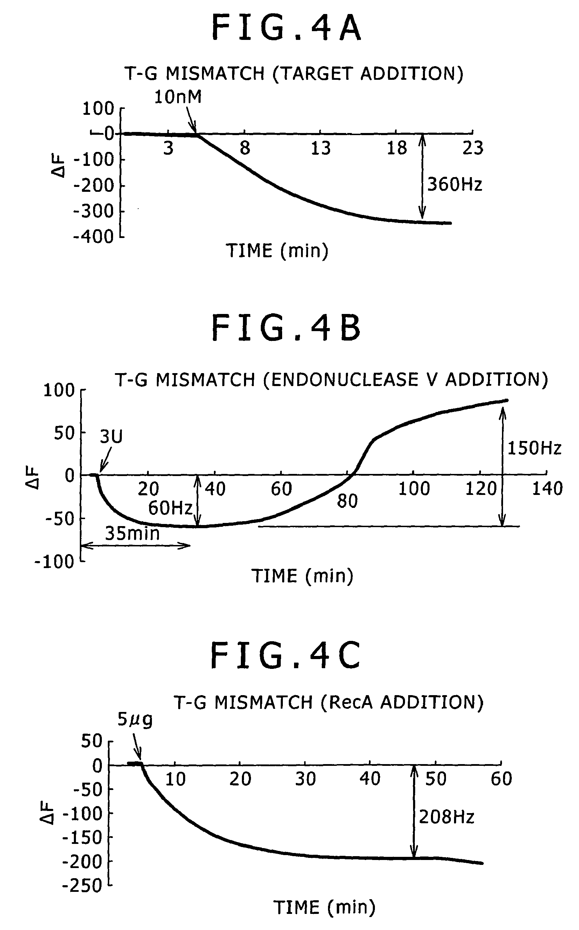

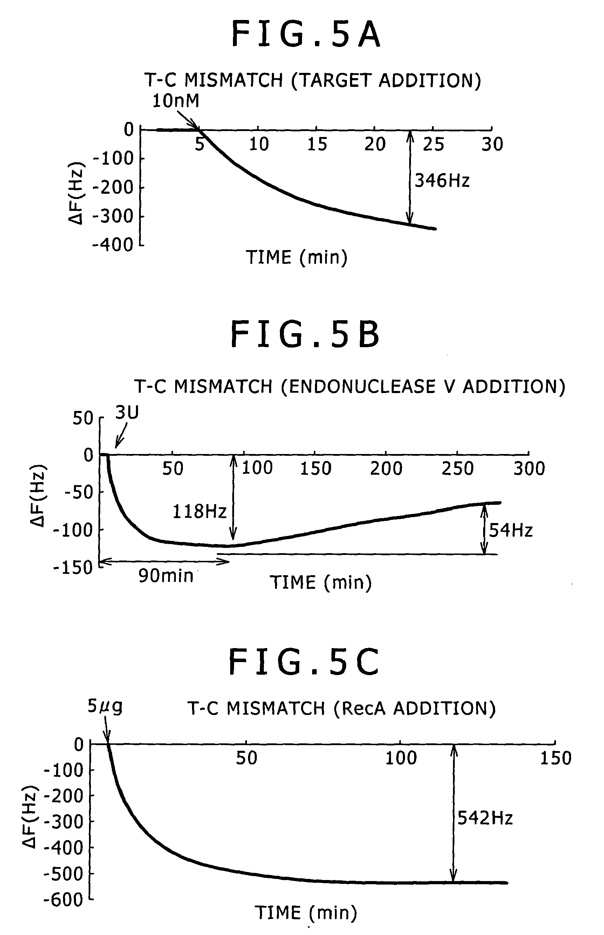

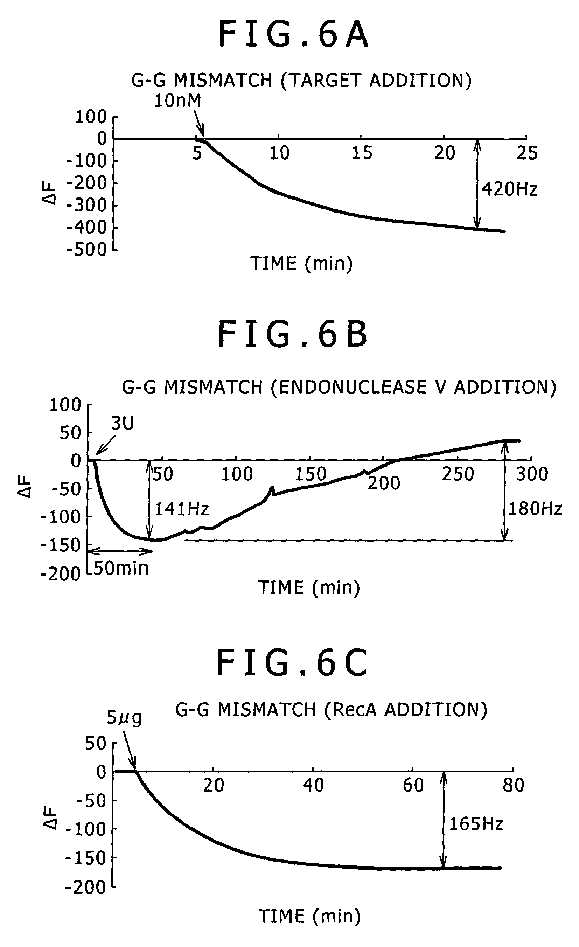

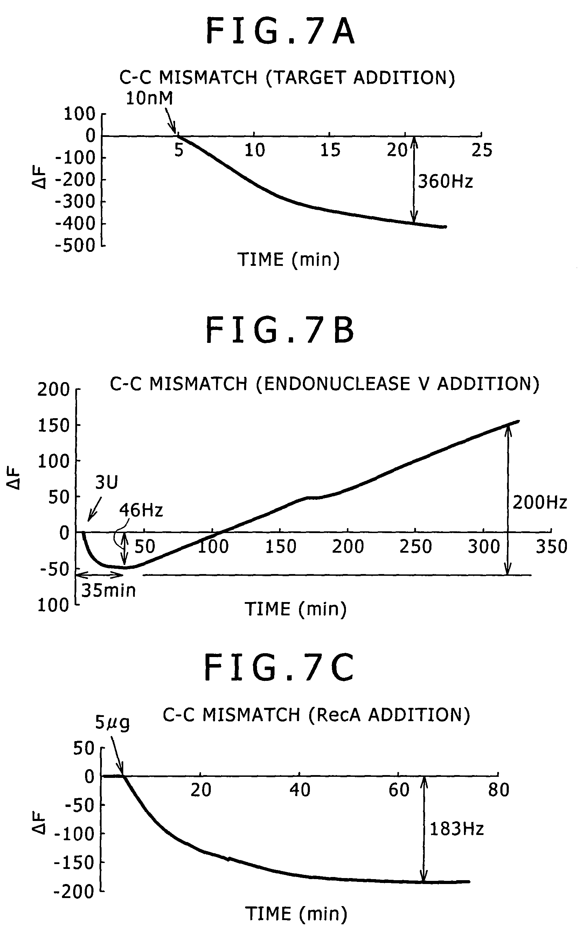

FIGS. 1A to 1C shows the results when (perfect match) nucleic acids, base pairs of which were all complementary, were used; FIGS. 2A to 2C shows the results when the A-G mismatch nucleic acids were used; FIGS. 3A to 3C shows the results when the T-T mismatch nucleic acids were used; FIGS. 4A to 4C shows the results when the T-G mismatch nucleic acids were used; FIGS. 5A to 5C shows the results when the T-C mismatch nucleic acids were used; FIGS. 6A to 6C shows the results when the G-G mismatch nucleic acids were used; and FIGS. 7A to 7C shows the results when the C-C mismatch nucleic acids were used.

In FIG. 1A through FIG. 7A, each diagram the figure number of which is accompanied by "A" shows variations in the resonance frequency of the quartz resonator when the corresponding target nucleic acid was added to the reaction bath.

In each of these diagrams, the resonance frequency of the quartz resonator decreased with time by the addition of the corresponding target nucleic acid. These results indicate that the detection probe immobilized on the quartz resonator and the target nucleic acid were hybridized to result in a weight increase.

In FIG. 1B through FIG. 7B, each diagram the figure number of which is accompanied by "B" shows variations in the resonance frequency of the quartz resonator when "E. COLI ENDONECLEASE V" was added to the reaction bath after the above-described procedure.

In the diagram of FIG. 1B, the resonance frequency of the quartz resonator remained substantially unchanged after the addition of the enzyme. In each diagram the figure number of which is accompanied by "B" in FIGS. 2B through FIG. 7B, on the other hand, the resonance frequency of the quartz resonator increased with time after the enzyme treatment. These results indicate that, when a double-stranded nucleic acid containing a mismatch site therein is subjected to the enzyme treatment, the amount of the double-stranded nucleic acid decreases, thereby suggesting its denaturation into a single-stranded form with time.

In FIG. 1C through FIG. 7C, each diagram the figure number of which is accompanied by "C" shows variations in the resonance frequency of the quartz resonator when RecA was added to the reaction bath after the above-described procedure.

In the diagram of FIG. 1C, the resonance frequency of the quartz resonator remained substantially unchanged after the addition of RecA. In each diagram the figure number of which is accompanied by "C" in FIGS. 2C through FIG. 7C, on the other hand, the resonance frequency of the quartz resonator increased with time by the addition of RecA after the enzyme treatment. These results indicate that RecA bound to the single-stranded nucleic acid existing on the quartz resonator, resulting in a weight increase. These results, therefore, indicate that, when a double-stranded nucleic acid containing a mismatch site therein was subjected to the enzyme treatment, the mismatch nucleic acid denatured into a single-stranded form with time.

The above-described results strongly suggest that in the case of a mismatched double-stranded nucleic acid, the cleavage of the mismatched double-stranded nucleic acid at the sites of mismatches with an enzyme, which recognize mismatch sites, induces site-specific cleavage at all single-base mismatches and subsequent to the cleavage by the enzyme, the double-stranded nucleic acid is denatured (dissociated) into a single-stranded form.

These results, therefore, suggest that by conducting the above-described respective treatments such as the enzyme treatment while using, for example, four detection probes different in the base species of SNP sites thereof, only the detection probe which is complementary at the SNP site can be detected.

FIG. 8 shows the results when A-A mismatch nucleic acids were used, FIG. 9 shows the results when A-C mismatch nucleic acids were used, and FIG. 10 shows the results when G-T mismatch nucleic acids were used.

FIG. 8 shows the results when the temperature of the reaction bath was set at 25°C, while FIG. 9 and FIG. 10 show the results when the temperatures of the reaction baths were set at 37°C. Further, FIG. 8 and FIG. 9 show the results when the mismatch sites were set at the seventh sites from the tops (the ends on the sides opposite to the immobilized ends), and FIG. 10 shows the results when the mismatch site was set at the seventh site from the bottom (the end on the side opposite to the immobilized end).

In FIG. 8, the resonance frequency of the quartz resonator decreased by 360 Hz by the addition of the detection probe ("Probe", 10 nM) and the target nucleic acid ("Target", 10 nM), and the resonance frequency of the quartz resonator decreased further by 17.0 Hz by the addition of the "E. COLI ENDONUCLEASE V". About 30 minutes later, however, the resonance frequency began to increase, and upon an elapse of 150 minutes, the resonance frequency increased by about 29.0 Hz.

In FIG. 9, the resonance frequency of the quartz resonator decreased by 100 Hz by the addition of the detection probe ("Probe", 10 nM), the resonance frequency of the quartz resonator decreased further by 145 Hz by the addition of the target nucleic acid ("Target", 10 nM), and the resonance frequency of the quartz resonator decreased still further by 20 Hz by the addition of "E. COLI ENDONUCLEASE V". About six minutes later, however, the resonance frequency began to increase, and upon an elapse of 150 minutes, the resonance frequency increased by about 151 Hz.

In FIG. 10, the resonance frequency of the quartz resonator decreased by 403 Hz by the addition of the detection probe ("Probe", 10 nM) and the target nucleic acid ("Target", 10 nM), and the resonance frequency of the quartz resonator decreased further by 46 Hz by the addition of the "E. COLI ENDONUCLEASE V". After that, however, the resonance frequency remained substantially unchanged.

Comparing FIG. 8 with FIG. 9, about 30 minutes elapsed in FIG. 8 until the resonance frequency began to increase after the addition of "E. COLI ENDONUCLEASE V", while in FIG. 9, the resonance frequency began to increase in about six minutes. These results indicate that a double-stranded nucleic acid, which has been cleaved at a mismatch site thereof by enzyme treatment, can be denatured into a single-stranded form in a shorter time by applying temperature treatment.

Comparing FIG. 9 and FIG. 10, the amount of the nucleic acid that denatured into a single-stranded form increased with time in FIG. 9, while substantially no nucleic acid denatured into a single-stranded form in FIG. 10. These results indicate that, when the site of an SNP is excessively close to an immobilized end, an enzyme capable of specifically cleaving single-stranded nucleic acid can no longer recognize the mismatch at the site of the SNP, and also that the site of an SNP in a detection probe can preferably be at a position 20 to 35% apart from the end opposite to the immobilized end.

### Example 2

In Example 2, an investigation was conducted as to whether or not Tm of a detection probe would be adjustable by chemically modifying it at a part thereof.

A detection probe and a target probe were provided firstly. The sequence of the detection probe is shown as SEQ ID NO: 21, while the sequence of the target probe is shown as SEQ ID NO: 22. The cytosines at the sixth, 14^{th} and 15^{th} from the 5'end of the detection probe were next methylated. Tm of the detection probe was then measured with the parts of its sequence methylated and unmethylated, respectively.

The results are shown in Table 1.

**Table 1**

| | Tm (°C) |
|---|---|
| Without methylation | 66.4 |
| With methylation | 67.6 |

As shown in Table 1, Tm increased with methylation compared to without methylation.

These results suggest that Tm of a detection probe can be adjusted by chemically modifying it at a part thereof.

It should be understood by those skilled in the art that various modifications, combinations, sub-combinations and alterations may occur depending on design requirements and other factor in so far as they are within the scope of the appended claims or the equivalents thereof.

## Claims

1. An SNP discrimination assay for discriminating base species of an SNP in a specific gene in a sample, which comprises the steps of:
hybridizing four detection probes, which have at least a flanking sequence around an SNP in said gene and are different in base species at a site of said SNP, with a target nucleic acid in said sample, said target nucleic acid having at least said flanking sequence around said SN in said gene, respectively;
cleaving three of four double-stranded nucleic acids, which have been formed by said hybridization step and contain noncomplementary base pairs, respectively, at sites of said noncomplementary base pairs;
denaturing only said three double-stranded nucleic acids, which have been cleaved at said sites of said noncomplementary base pairs, into single-stranded forms; and
detecting one of said four detection probes, said one detection probe still retaining a double-stranded form after said nucleic acid denaturation step.

2. The SNP discrimination assay according to claim 1, wherein in said mismatch site cleavage step, said three double-stranded nucleic acids, which contain said noncomplementary base pairs, respectively, are cleaved at said sites of said noncomplementary base pairs by using an enzyme capable of specifically cleaving single-stranded nucleic acid.

3. The SNP discrimination assay according to claim 2, wherein said enzyme is an enzyme capable of cleaving phosphate backbones at mismatch sites.

4. The SNP discrimination assay according to claim 1, wherein in said nucleic acid denaturation step, only said three double-stranded nucleic acids which have been cleaved at said sites of said noncomplementary base pairs are denatured into said single-stranded forms by making use of differences in melting temperature between remaining one of said four double-stranded nucleic acids, said remaining one double-stranded nucleic acid having not been cleaved in said mismatch site cleavage step, and said three double-stranded nucleic acids cleaved at said sites of said noncomplementary base pairs in said mismatch site cleavage step.

5. The SNP discrimination assay according to claim 1, wherein in said base species detection step, an intercalator capable of specifically binding to double-stranded nucleic acids is used.

6. The SNP discrimination assay according to claim 1, wherein at least one of said detection probes has been artificially modified at a part of a sequence thereof to adjust a melting temperature thereof.

7. The SNP discrimination assay according to claim 1, further comprising the step of, before said hybridization step, artificially modifying a part of a sequence of said target nucleic acid to adjust a melting temperature thereof.

8. A DNA chip for SNP discrimination, said DNA chip having a substrate with plural reaction regions formed as spots for hybridization on a surface of said substrate, comprising:
four detection probes, which have at least a flanking sequence around an SNP in a specific gene, are different in base species at a site of said SNP, and are separately held in different ones of said reaction regions.

9. The DNA chip according to claim 8, wherein at least one of said detection probes has been artificially modified at a part of a sequence thereof to adjust a melting temperature thereof.
